Europäisches Patentamt

European Patent Office

Office européen des brevets

(11) Publication number: **0 138 242**
**A1**

(12) **EUROPEAN PATENT APPLICATION**

(21) Application number: **84201220.5**

(22) Date of filing: **27.08.84**

(51) Int. Cl.⁴: **C 12 N 15/00,** C 12 P 21/00,
A 61 K 39/215

---

(30) Priority: **07.09.83 NL 8303101**

(43) Date of publication of application: **24.04.85**
**Bulletin 85/17**

(84) Designated Contracting States: **AT BE CH DE FR GB IT
LI LU NL SE**

(71) Applicant: **DUPHAR INTERNATIONAL RESEARCH B.V,
C.J. van Houtenlaan 36, NL-1381 CP Weesp (NL)**

(72) Inventor: **van der Zeijst, Bernardus, c/o
OCTROOIBUREAU ZOAN B.V. Apollolaan 151,
NL-1077 AR Amsterdam (NL)**
Inventor: **Bredenbeek, Petrus J., c/o OCTROOIBUREAU
ZOAN B.V. Apollolaan 151, NL-1077 AR Amsterdam (NL)**

(74) Representative: **Muis, Maarten et al, OCTROOIBUREAU
ZOAN B.V. Apollolaan 151, NL-1077 AR Amsterdam (NL)**

(54) Method of preparing vaccines against coronaviruses.

(57) The invention relates to a new and fast method of preparing synthetic DNA's which correspond to genome RNA or to messenger RNA's (m RNA's) of coronaviruses.

The proteins obtained after expression of the so obtained synthetic DNA's can be used for the preparation of vaccines.

According to this new method among others vaccines against infectious bronchitis virus, feline infectious peritonitis virus, and transmitable gastroenteritis virus can be obtained.

EP 0 138 242 A1

Method of preparing vaccines against coronaviruses.

The invention relates to a new and fast method of preparing synthetic DNA's which correspond to genomic RNA or to messenger RNA's (mRNA's) of coronaviruses. The DNA molecule obtained according to the method of the invention can be introduced into a cloning vector which is capable of bringing DNA molecules to expression after the cloning vector has been brought in a suitable host cell by transformation. The product (protein) obtained after expression may be used for the preparation of a vaccine which stimulates the formation of antibodies or a cellular immune response against coronaviruses.

Another application of the method according to the invention is that the nucleotide sequences of the viral RNA's can be determined from the DNA clones, after which synthetic oligopeptide vaccines which provide protection against diseases caused by coronaviruses can be prepared by means of the knowledge of said nucleotide sequences.

The method of the invention may also be used for the direct determination of the nucleotide sequence of viral RNA's by using a DNA primer which binds to the RNA. Said primer then serves as a starting-point for the sequencing reaction.

Up till now, virus vaccines have been prepared on the basis of either attenuated live virus, or inactivated virus. Such vaccines comprises the whole virus particle.

It has further been tried to prepare a so-called sub-unit vaccine from a few viruses. Such vaccines do not comprises the whole virus particle, but only one or a few immunogenically active virus proteins.

The latest development in the field of the vaccine preparation is the use of recombinant DNA techniques. DNA molecules which comprise completely or partly the nucleotide sequence of the virus genome, can be introduced into a cloning vector. When such cloning vectors are introduced into a suitable host cell by transformation, the virus DNA can come to expression. The proteins to be obtained in this manner may be used for the preparation of vaccines.

Copy DNA (cDNA) molecules from positive stranded RNA viruses have so far been obtained by initiating ("priming") the reaction with oligo (dT) which binds to the poly (A) stretch located at the 3'-end of the virion RNA or of the mRNA's.

By means of this known technique it is easier to obtain recombinant DNA clones which correspond to the 3'-end of the genome than with the 5'-end. That this is true may appear from the fact that for the most intensively studied coronaviruses, i.e. mouse hepatitis virus strain A 59 (MHV-A59), so far only the nucleotide sequence of genes coding for the N and $E_1$ protein, which are located at the extreme 3'-end of the genome have been described. The genetic information which codes for the $E_2$ molecules which elicite neutralising antibodies, is much more difficult to clone and sequence.

A method has now been found by means of which synthetic cDNA's which correspond to genome RNA or mRNA's of coronaviruses can rapidly be obtained.

Recent research has shown that the 6 subgenomic mRNA's of mouse hepatities virus (MHV) which are synthesized together with the positive stranded genome in the cyto plasma of the host cells are composed of a leader sequence and a body sequence which are joined together by a mechanism which is analogous to, but differs clearly from, the mechanism found in conventional RNA splicing. This
mechanism involves that non-contiguous sequences are
joined to produce functional mRNA in the cytoplasma of
coronavirus-infected cells.

Corona viruses are eveloped positive stranded RNA viruses.
The genome RNA is linear and 15,000 - 20,000 bases in
length. Of the corona viruses, MHV-strain A59 has been
studied most intensively. MHV replicates in the cell cytoplasma and viral genetic information is expressed in infected cells as one genome RNA and several sub-genomic
mRNA's. These mRNA's are formed in non-equimolar amounts
but in a relatively constant proportion during the infection period (see J.L. Leibowitz et al., Virology $\underline{114}$
(1981), $\underline{pp}$. 39-51). By analysis of oligonucleotides ob-
tained after RNase $T_1$ digestion of viral RNA it was
found that the $T_1$ oligonucleotides 10 and 19 from mRNA7
of the MHV strain A59 (for the nomenclature used see
M.M.C. Lai et al, J.Virol. $\underline{41}$ (1982), $\underline{pp}$. 557-565) are not
present in the corresponding 3'-end of the genome (see
W.J.M. Spaan et al, J.Virol. $\underline{42}$ (1982), $\underline{pp}$. 432-439). This
might be an indication that these oligonucleotides are
derived from a leader sequence which all mRNA's have in
common. Oligonucleotide 10 was found in the larger mRNA's
and in genome RNA, but for oligonucleotide 19, mRNA specific electrophoretic mobility differences could be demonstrated. This oligonucleotide is present in mRNA7, mRNA6
and mRNA5 as oligonucleotide 19, 19$\underline{a}$ and 3$\underline{a}$, respectively.
The oligonucleotides 19 and 19$\underline{a}$ have a strongly resembling
base composition. Oligonucleotide 17 which has been found
in mRNA6 and in larger mRNA's, but no in mRNA7, also has a
base composition which resembles that of oligonucleotides
19 and 19$\underline{a}$ (see J. Armstrong et al, Nucl.Acids Res. $\underline{11}$
(1983), $\underline{pp}$. 883-891). At the 5'-end the mRNA's have at
least 5 nucleotides in common with each other.

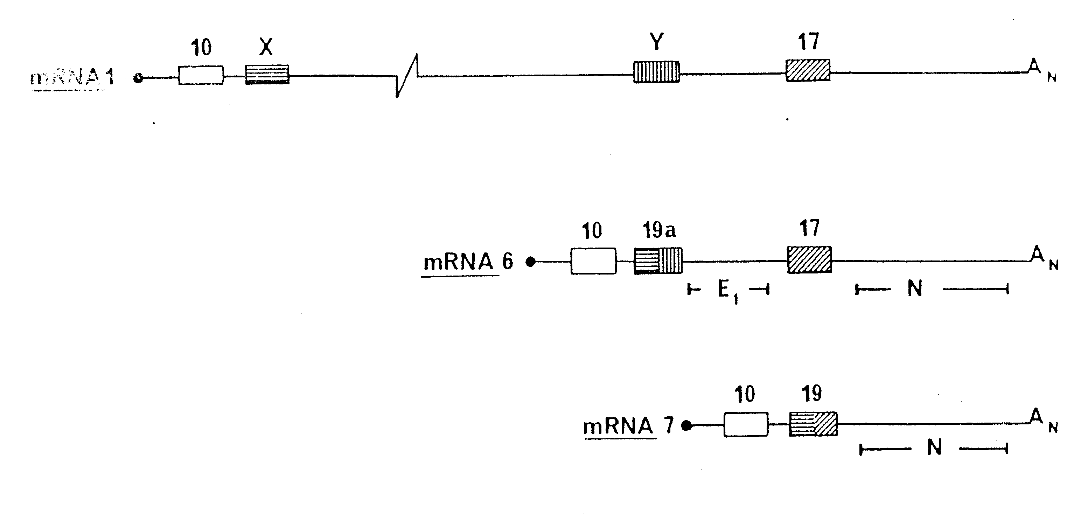

These data can be represented in the model of Figure 1. In Figure 1 the symbols ● and $A_N$ represent the 5' terminal structures and the 3' terminal polyadenylate structures, respectively. RNase $T_1$ resistant oligonucleotides are indicated by numbers. The sequences X and Y have not yet been identified. $E_1$ (matrix/envelope protein) and N (nucleocapside protein) are the translation products of mRNA6 and 7, respectively. The boxed regions are represented on a larger scale than the other region of the mRNA's.

In the model of Figure 1 the sequences which are present at the 5'-end of genome RNA (or mRNA1) have also to be present at the 5'-end of each sub-genomic mRNA. These sequences will hereinafter be referred to as "leaders". Based on the above findings, MHV-A59 nucleotide 10 will have to be present in these sequences. A part of the MHV--A59 oligonucleotides 19 and 19a will also have to be present within the leader, but differences will arise from fusion of the leader sequences with the various "bodies" of the mRNA's. Oligonucleotide 17 will represent sequences at the 5'-end of the mRNA7 body, part of which is lost in building up mRNA7 but not, for example, in that of mRNA6.

The model of Figure 1 was tested by means of electronmicroscopic analysis of hybrids which are formed between single-stranded cDNA copied from mRNA7 with genomic RNA or mRNA6. The data obtained in this manner strongly support the model of Figure 1.

The model of Figure 1 is also confirmed by the differences found in the nucleotide sequence of the region immediately upstream of the nucleocapside gene present both in mRNA7 and in the genome.

Further support for the Figure 1 model was found by direct determination of the sequence at the 5'-end of mRNA7 which has to be indentical to the correspoding region of the genome of MHV-A59.

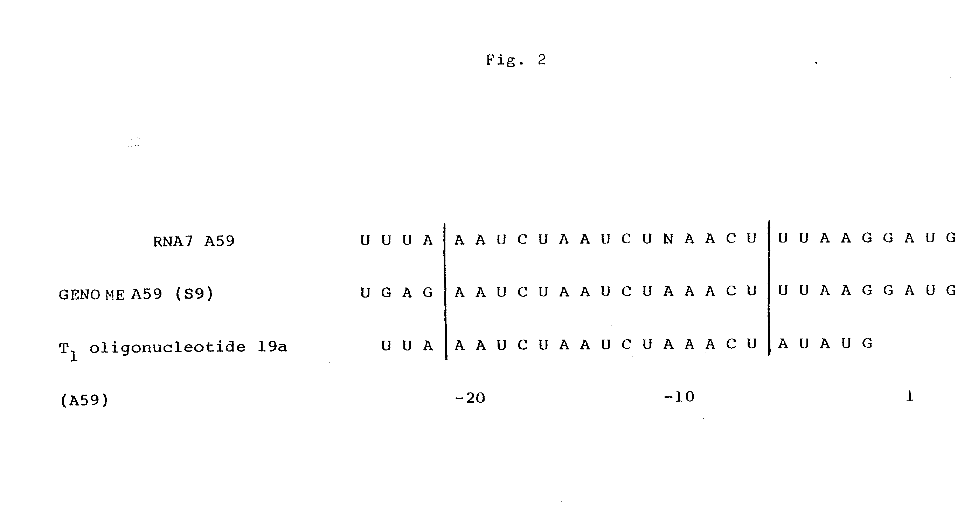

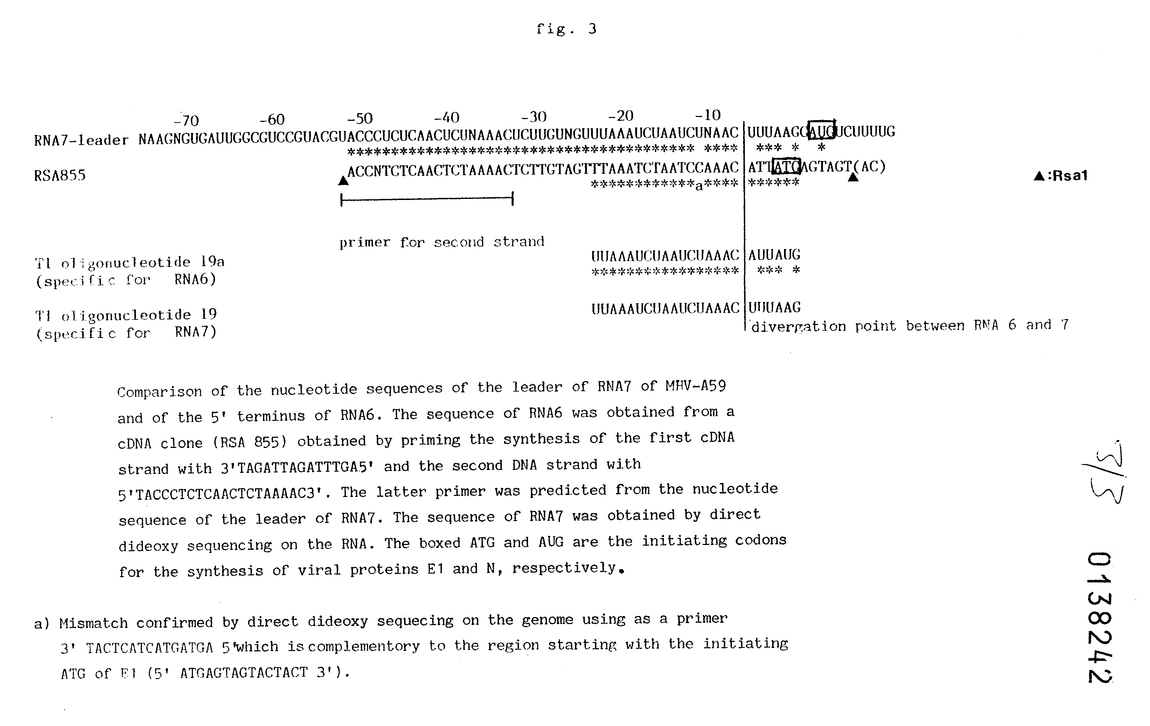

The above-described findings are summarized in Figures 2 and 3. Figure 2 relates to the location of the fusion site of "body" and "leader" of MHV A59 mRNA's. The sequences were determined as follows:

- RNA7 by direct sequencing on the RNA with a DNA primer;
- genome (mRNA1) by sequencing of a recombinant cDNA-clone (S9);
- oligonucleotide 19a by looking for maximum homology with the two preceding sequences.

From this it will be clear that the 5'-end of the MHV-A59 mRNA7 body cannot extend beyond position-21, the site of divergence of mRNA7 and the genome. As stated above, $T_1$ oligonuclotide 19a (which is specific for mRNA6) and $T_1$ oligonucleotide 19 (which is specific form mRNA7) have a very similar base composition. By predicting the sequence of 19a from the base composition (see M.M.C. Lai et al, J.Virol. 41 (1982), pp.557-565), and comparison with the MHV-A59 sequence between the positions -24 and-2 (Figure 2) it can be seen that the 3'-terminus of the leader sequence fused to mRNA6 cannot extend beyond the first base difference (read from 5' to 3') in oligonucleotides 19 and 19a. From this it follows that for MHV-A59 the fusion of leader and body sequences producing the oligonucleotides 19 and 19a takes place somewhere within the sequence 5'AAUCUAAUCUAAACU 3'. Hence everthing indicates that the repeating palindrome AUCUA (or the sequence AAUCU) during the fusion process serves as a recognition signal.

The available data can be explained by means of a polymerase jumping mechanism (see J.Perrault, Curr.Top.Microbiol.Immunol. 93 (1981), 151-207). Such a mechanism involves the synthesis of a short RNA transcript from the 3'-end of the negatively stranded "template". The polymerase/leader complex or the leader alone is then translocated to an internal position on the negatively stranded template where the transcription is resumed. This translocation may or may not involve dissociation of the leader from the template.

The above-described mechanism is surprising not only from a scientific point of view, but it also opens up new routes for the preparation of cDNA molecules of specific genes, and for determining the sequence of these genes. This will be demonstrated with reference to MHV-A59 as an example. As has been demonstrated hereinbefore, the fusion between the leader and the body sequence takes place in the sequence 5'AAUCUAAUCUAAACU 3'. This sequence is present in the internal region of the genome between gene F and gene G. The invention is also based on the fact that this sequence may be assumed to be present also, either completely or partly in the other intergene regions. This menas that a primer molecule which is complementary to this sequence or a part thereof (for example, 3'TAGATTAGATTTGA 5') may be used as a starting point for the preparation of cDNA or for establishing the sequence thereof. When genome RNA is used, this will result in the synthesis of a number of discrete single-stranded cDNA molecules having dimensions which are characteristic of the gene to which they correspond. The correct DNA molecule can be selected, after which the second strand can be synthesised and cloned.

It is also possible to bind the same primer to a purified mRNA (1 to 7). When the correct concentration is used, this will bind in an internal position, but not in the leader, so that among others single-stranded cDNA molecules are obtained corresponding to the region of the mRNA which is not present in the next following smaller mRNA. This cDNA molecule can be isolated and a second strand may be made by "priming" with a nucleotide which corresponds to RNase $T_1$ oligonucleotide 10 (5'TACCCTCTCAACTCTAAAAC 3') or a similar molecule from that region.

The invention will now be described in greater detail with
reference to the ensuing specific examples.


EXAMPLE I


RNA6 was isolated from cells infected with MHV-A59 as
described by W.J.M. Spaan et al in J.Virol. 42 (1983),
422-439 and used as a matrix for the synthesis of cDNA.
The above-mentioned primer 3'TAGATTAGATTTGA 5' was used as
a starting-point for the first DNA strain. The reaction
mixture (10 ul) contained 1 ug of RNA, 50 ng of primer and
in addition 50 mM of Tris-HCl (pH 8.3), 50 mM of KCl, 8 mM
of $MgCl_2$, 1mM of dithiothreitol, 2 units/ug of RNA reverse transcriptase (J.W. Beard, J.Virol. 29, 517-522
(1979), 500 uM each of dCTP, dGTP, and TTP, 250 uM of dATP
and 5-10 uCi of alpha-$^{32}$P-dATP (The Radiochemical Centre
Amersham, England; > 400 Ci/mmmol). The mixture was then
incubated at 42°C for 30 minutes. The cDNA formed was analysed via gel electrophoresis. There was a major product
of the expected size (approximately 770 bases).


In order to be able to monitor the synthesis of the second
DNA strand, the experiment was repeated with the following
concentrations of desoxynucleotide triphosphonates in the
reaction mixture; 500 uM of dATP, dGTP and TTP each, 200
uM of dCTP and 25 uCi of $^{3}$H-dCTP (21 Ci/mmol; the Radiochemical Centre Amersham, England). After the synthesis of
the first cDNA strand, the RNA template was hydroxlyzed by
alkaline treatment. 0.5 M EDTA (pH 8.0) and 150 mM of NaOH
were added successively to the reaction mixture until a
final concentration of 20 mM and 50 mM, respectively.
After an incubation at 65°C for 1 hour and at 37°C for 8
hours, the mixture was neutralised and the cDNA was
re-isolated by extraction with phenol-chloroform as
described by T.Maniatis et al in Molecular Cloning, A Laboratory Manual, Cold Spring Harbor Laboratory 1982, p.

233. The composition of the reaction mixture for the synthesis of the second DNA strand (15 ul) was 100 mM of Hepes-KOH pH 6.9, 50 mM of KCl, 4 mM of $MgCl_2$, 0.5 mM of DTT, 500 uM of dCTP, dGTP and TTP each, 250 uM of dATP, 5-10 uCi of alpha-$^{32}$P-dATP (The Radiochemical Centre, Amersham, England; > 400 Ci/mmol), 0.7 units of cDNA-polymerase 1 (Klenow enzyme, Boehringer Mannheim), 10 ng of cDNA to RNA6 and 100 ng of the primer 5'TACCCTCTCAACTCTAAAAC 3'. Before the addition of the heat-labile components, the reaction mixture was heated to 96°C for 10 minutes and then slowly cooled to stimulate the bonding of the DNA-primer. After starting the reaction, incubation was carried out at 22°C for 2 hours. The formed 2nd cDNA strand was analyzed by means of gel electrophoresis and proved to co-migrate with the first cDNA strand.

The conclusion is that RNA6 indeed comprises at its 5'-end a sequence identical to the second-strand primer. So this part must be identical to the 5'-end of RNA7 on the guidance of which the primer was synthesized. Hence it is possible by means of the second-strand-primer to initiate the second cDNA strand of sub-genomic messenger RNA's other than RNA7.

EXAMPLE II

Using the same method as described above a larger quantity of double-stranded (ds) cDNA to RNA6 was prepared. For this purpose, instead of purified RNA6 so-called poly(A)$^+$RNA's were used, prepared from Sac(-) cells infected with MHV-A59. This RNA preparation contains all viral mRNA's which in infected cells constitute approximately 5% of the total RNA in the cell. It was purified via chromatography over oligo (dT)-cellulose. (T. Maniatis et al, Molecular Cloning, A Laboratory Manual, Cold Spring Harbor

Laboratory, p. 197-198). The double-stranded cDNA comprised, in addition to the above-described products, also
larger molecules, probably originating from the other
mRNA's. Without a second-strand-primer no detectable DNA
synthesis took place. After digestion (T. Maniatis et al,
Molecular Cloning, A Laboratory Manual, Cold Spring Harbor
Laboratory, p. 104-106) of portions of the cDNA with the
restriction enzymes RsaI or FnuDII the restriction fragments were cloned in the "SmaI site" of the replicative
form of M13 mp9 DNA. Fragments obtained after digestion of
cDNA with the restriction enzyme HpaII were cloned in the
"AccI site" of the replicative form of M13 mp8 (J. Messing, Genetic Engineering: Principles and Methods (1982),
Vol IV, 19-36, edited by J.K. Setlow and A. Hollaender,
Plenum Press, New York). The fragments were ligated to the
vector in a reaction mixture (15 ul) containing 50 mM of
Tris-HCl (pH 7.5), 10 mM of $MgCl_2$, 0.5 mM of ATP, 10 mM
of dithiothreitol, 50 ug/ml of BSA, 1 mM of spermidine, 20
ng of vector (linearized by SmaI or AccI), and 35 ng of
cDNA digest. For "sticky end" ligation (HpaII), 40 New
England Biolabs units of ligase (New England Biolabs,
Beverly, Massachusetts, USA) were used per reaction, for
"blunt end" ligation (RsaI, FnuDII) 400 units of ligase
were used per reaction. Ligase reactions were carried out
overnight at 4°C. Subsequently competent Escherichia coli
K12 (JM 103) cells were transfected with the ligase reaction mixture. Lac (-) plaques (3-11% of the overall number
of plaques) were isolated, and phage was grown up (F.
Sanger et al, J.Mol.Biol., 145 (1980), 161-178), from
which viral DNA was isolated; after isolation phage DNA
was used immediately for dideoxy sequence analysis of the
cDNA insert using a single-stranded DNA sequence (P.L.
Biochemicals, Inc. Milwaukee, Wisconsin, USA). Notably the
sequence of the DNA clone RSA855 is relevant. This is
shown in Figure 3 and compared with the corresponding leader sequence in RNA7. The data are in complete agreement with Example I and demonstrate that priming of the synthesis of the second strand of cDNA can indeed take place in
the manner invented by means of the so-called second-
-strand primer 5'TACCCTCTCAACTCTAAAAC 3'.

## CLAIMS

1. A method of preparing synthetic cDNA's or for the determination of nucleotide sequences of coronaviruses, characterized in that cDNA's are prepared which correspond to genome RNA or mRNA's with the use of a primer for the synthesis of the first cDNA-strand which binds to the repeating intergenic sequences in the genome RNA or the mRNA's, and, if desired, using as a starting point a primer for the synthesis of the second cDNA-strand which is homolgous to a part of the "leader" occurring in each of the RNA's.

2. A method as claimed in Claim 1, characaterized in that 3'TAGATTAGATTTGA 5' is used as a first-strand-primer and 5'TACCCTCTCAACTCTAAAAC 3' is used as a second-stand--primer.

3. A method as claimed in Claim 1 or 2, characterized in that cDNA's of the mous-hepatitis virus strain A59 are prepared.

4. A method as claimed in claims 1-3, characterized in that cDNA's of the mouse-hepatitis virus strain A59 are prepared.

5. A method as claimed in Claim 1, characterized in that cDNA's of the coronoviruses infectious bronchitis (IB)-virus, feline infectious peritonitis (FIP)-virus or transmitable gastroenteritis (TGE)-virus are prepared.

6. cDNA's obtained by means of the method as claimed in Claims 1-5.

7. A method of preparing coronovirus proteins, characterized in that peplomer proteins and/or matrix/envelope proteins, or parts thereof are prepared by using the method as claimed in Claims 1-5.

8. Proteins or parts thereof (oligopeptides) obtained by means of the method as claimed in Claim 7.

9. A vaccine comprising at least one protein or oligopeptide of Claim 8.

Fig. 2

| | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|

RNA7 A59     U U U A | A A U C U A A U C U N A A C U | U U A A G G A U G

GENOME A59 (S9)     U G A G | A A U C U A A U C U A A A C U | U U A A G G A U G

$T_1$ oligonucleotide 19a     U U A | A A U C U A A U C U A A A C U | A U A U G

(A59)                 -20             -10             1

2/3

0138242

fig. 3

```
              -70        -60        -50        -40        -30        -20        -10
RNA7-leader NAAGNGUGAUUGGCGUCCGUACGUACCCUCUCAACUCUNAAACUCUUGUGNGUUUAAAUCUAAUCUNAAC|UUUAAGCAUGUCUUUUG
                                   ***********************************  ****|*** *  *
RSA855                             ▲ACCNTCTCAACTCTAAAACTCTTGTAGTTTAAATCTAATCCAAAC|ATTATGAGTAGT(AC)                 ▲:Rsa1
                                                                      **********a****|******         ▲
                                   ├─────────────────────────────┤

                                   primer for second strand
                                                                   UUAAAUCUAAUCUAAAC|AUUAUG
Tl oligonucleotide 19a                                             ***************** |*** *
(specific for   RNA6)

Tl oligonucleotide 19                                              UUAAAUCUAAUCUAAAC|UUUAAG
(specific for   RNA7)                                                               |divergation point between RNA 6 and 7
```

Comparison of the nucleotide sequences of the leader of RNA7 of MHV-A59
and of the 5' terminus of RNA6. The sequence of RNA6 was obtained from a
cDNA clone (RSA 855) obtained by priming the synthesis of the first cDNA
strand with 3'TAGATTAGATTTGA5' and the second DNA strand with
5'TACCCTCTCAACTCTAAAAC3'. The latter primer was predicted from the nucleotide
sequence of the leader of RNA7. The sequence of RNA7 was obtained by direct
dideoxy sequencing on the RNA. The boxed ATG and AUG are the initiating codons
for the synthesis of viral proteins E1 and N, respectively.

a) Mismatch confirmed by direct dideoxy sequecing on the genome using as a primer
   3' TACTCATCATGATGA 5'which is complementory to the region starting with the initiating
   ATG of E1 (5' ATGAGTAGTACTACT 3').

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 94, no. 21, 25th May 1981, page 256, no. 169619k, Columbus, Ohio, USA; P.J.M. ROTTIER et al.: "Translation of three mouse hepatitis virus strain A59 subgenomic RNAs in Xenopus laevis oocytes" & J. VIROL. 1981, 38(1), 20-26 * Abstract * | 8 | C 12 N 15/00 C 12 P 21/00 A 61 K 39/215 |
| | --- | | |
| D,A | NUCLEIC ACIDS RESEARCH, vol. 11, no. 3, 11th February 1983, pages 883-891, IRL Press Ltd., Oxford, GB; J. ARMSTRONG et al.: "Sequence of the nucleocapsid gene from murine coronavirus MHV-A59" | | |
| | --- | | **TECHNICAL FIELDS SEARCHED (Int. Cl.4)** |
| D,A | JOURNAL OF VIROLOGY, vol. 42, no. 2, May 1982, pages 432-439; W.J.M. SPAAN et al.: "Sequence relationships between the genome and the intracellular RNA species 1,3,6, and 7 of mouse hepatitis virus strain A59" | | C 12 N C 12 P A 61 K |
| | --- | | |
| D,A | JOURNAL OF VIROLOGY, vol. 41, no. 2, February 1982, pages 557-565; M.M.C. LAI et al.: "Further characterization of mRNA's of mouse hepatitis virus: Presence of common 5'-end nucleotides" | | |
| | --- -/- | | |

The present search report has been drawn up for all claims

| Place of search THE HAGUE | Date of completion of the search 02-01-1985 | Examiner DESCAMPS J.A. |
|---|---|---|

European Patent
Office

| | DOCUMENTS CONSIDERED TO BE RELEVANT | | Page 2 | |
|---|---|---|---|---|
| Category | Citation of document with indication, where appropriate, of relevant passages | Relevant to claim | CLASSIFICATION OF THE APPLICATION (Int. Cl.4) | |
| A | NUCLEIC ACIDS RESEARCH, vol. 11, no. 15, 11th August 1983, pages 5045-5054, IRL Press Ltd., Oxford, GB; M.A. SKINNER et al.: "Coronavirus JHM: nucleotide sequence of the mRNA that encodes nucleocapsid protein" | | | |
| | ----- | | | |
| | | | TECHNICAL FIELDS SEARCHED (Int. Cl.4) | |

The present search report has been drawn up for all claims

| Place of search THE HAGUE | Date of completion of the search 02-01-1985 | Examiner DESCAMPS J.A. |
|---|---|---|

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO Form 1503. 03.82